(19) Europäisches Patentamt
European Patent Office
Office européen des brevets

(11) EP 3 500 238 B1

(12) **EUROPÄISCHE PATENTSCHRIFT**

(45) Veröffentlichungstag und Bekanntmachung des Hinweises auf die Patenterteilung:
**07.12.2022 Patentblatt 2022/49**

(21) Anmeldenummer: **17734254.0**

(22) Anmeldetag: **14.06.2017**

(51) Internationale Patentklassifikation (IPC):
***A61K 8/362*** *(2006.01)* ***A61K 8/19*** *(2006.01)*
***A61Q 5/00*** *(2006.01)*

(52) Gemeinsame Patentklassifikation (CPC):
**A61K 8/362; A61K 8/19; A61Q 5/004**

(86) Internationale Anmeldenummer:
**PCT/EP2017/064495**

(87) Internationale Veröffentlichungsnummer:
**WO 2018/033272 (22.02.2018 Gazette 2018/08)**

(54) **FARBSCHUTZ-HAARBEHANDLUNGSMITTEL MIT ERDALKALISALZEN VON DICARBONSÄUREN**

COLOR PROTECTION HAIR TREATMENT AGENT WITH EARTH ALKALINE SALTS OF DICARBOXYLIC ACIDS

PRODUITS DE SOIN CAPILLAIRE POUR CHEVEUX COLORÉS, CONTENANT DES SELS ALCALINO-TERREUX D'ACIDE DICARBOXYLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.08.2016 DE 102016215200**

(43) Veröffentlichungstag der Anmeldung:
**26.06.2019 Patentblatt 2019/26**

(73) Patentinhaber: **Henkel AG & Co. KGaA**
**40589 Düsseldorf (DE)**

(72) Erfinder:
- **KNÜBEL, Georg**
  **40219 Düsseldorf (DE)**
- **FÖRSTER, Thomas**
  **40591 Düsseldorf (DE)**

(56) Entgegenhaltungen:
**EP-A1- 3 042 940**
**WO-A1-97/30601**
**WO-A1-99/00109**
**WO-A1-2016/030353**
**DE-A1- 10 007 198**
**JP-A- 2009 007 283**

EP 3 500 238 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung liegt auf dem Gebiet der Kosmetik. Gegenstand der vorliegenden Erfindung sind Haarbehandlungsmittel zur Verringerung und/oder Vermeidung des Ausblutens und/oder Verblassens künstlich erzeugter Haarfarben, die auf einem wässrigen kosmetischen Träger basieren, einen pH-Wert im Bereich von 3,5 bis 6,0 besitzen und die mindestens 0,4 bis 2,6 Gew.-% Calciumsuccinat enthalten.

Ein weiterer Gegenstand der vorliegenden Erfindung ist ein Verfahren zur Verringerung und/oder Vermeidung des Ausblutens und/oder Verblassens künstlich erzeugter Haarfarben, bei welchem die Haare zunächst mit einem Färbemittel gefärbt und im Anschluss daran mit einem Farbschutzmittel des ersten Erfindungsgegenstands behandelt werden.

Ein weiterer Gegenstand der vorliegenden Erfindung ist eine Mehrkomponenten-Verpackungseinheit (Kit-of-Parts), welche getrennt voneinander konfektioniert in zwei Containern die Mittel (A) und (B) umfasst, wobei es sich bei dem Mittel (A) um ein Mittel zum Färben von Haaren handelt und das Mittel (B) ein Farbschutzmittel des ersten Erfindungsgegenstands ist.

Schließlich ist ein weiterer Gegenstand der vorliegenden Erfindung die Verwendung eines Mittels das einen wässrigen kosmetischen Träger umfasst, und(a) einen pH-Wert im Bereich von 3,5 bis 6,0 besitzt und(b) mindestens ein Erdalkalisalz einer Dicarbonsäure mit 2 bis 8 C-Atomen enthält. zum Farbschutz von gefärbten Haaren, bzw. die Verwendung des Mittels zur Verringerung und/oder Vermeidung des Ausblutens und/oder Verblassens künstlich erzeugter Haarfarben.

Menschliches Haar wird heute in vielfältiger Weise mit haarkosmetischen Zubereitungen behandelt.

Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Im Bereich der Haarfärbung sind im Wesentlichen zwei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. In vielen Fällen werden weiterhin zusätzlich direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Nach dem Färbeprozess ist das Haar über einen langen Zeitraum den verschiedensten Umweltbelastungen ausgesetzt. Diese reichen von der täglichen Bewitterung der Haare, beispielsweise durch Sonnenstrahlen und Haarwäschen, über mechanische, durch das Frisieren hervorgerufene Beanspruchungen bis zu chemischen Einflüssen, sofern der Konsument die Haare einem nachfolgenden Haarfärbe- oder Formungsprozess unterwirft. Die Umwelteinflüsse haben nicht nur auf die Haarstruktur selbst, sondern auch auf die nach dem Färbeprozess im Haar befindlichen Farbstoffe große Auswirkungen. Die Farbstoffe können durch die Belichtung ausbleichen, oder durch Schweiß oder Shampoonieren aus dem Haar heraus gewaschen werden. Das durch Shampoos oder Waschwasser bedingte Herauslösen der Farbstoffe aus der Haarfaser wird auch als Ausbluten bezeichnet. Der Fachmann fasst das Vermögen der Farbstoffe, all diesen Umwelteinflüssen zu widerstehen, auch unter dem Betriff Echtheitseigenschaften zusammen. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und unerwünschten Farbverschiebung kommen. Sowohl das Auftreten von Farbverschiebungen als auch insbesondere das durch Auswaschen bedingte Verblassen und Ausbluten der Färbungen ist vom Verbraucher unerwünscht.

Im Stand der Technik sind bereits verschiedene Farbschutzmittel bekannt. Diese sind jedoch noch verbesserungswürdig.

Die Aufgabe der vorliegenden Erfindung bestand daher in der Bereitstellung von Haarbehandlungsmitteln und -verfahren, mittels derer sich das Ausbluten und das Verblassen von künstlich erzeugten Haarfarben verringern oder nach Möglichkeit sogar komplett vermeiden lässt.

Überraschenderweise hat sich nun herausgestellt, dass künstlich gefärbte Haare optimal vor dem Verblassen und Ausbluten geschützt werden können, wenn diese mit einem wasserhaltigen Mittel behandelt werden, welches einen pH-Wert von 3,5 bis 6,0 besitzt und welches mindestens ein Erdalkalisalz einer Dicarbonsäure mit 2 bis 8 C-Atomen enthält.

Ein erster Gegenstand der vorliegenden Erfindung ist ein Haarbehandlungsmittel zur Verringerung und/oder

Vermeidung des Ausblutens und/oder Verblassens künstlich erzeugter Haarfarben, welches einen wässrigen kosmetischen Träger umfasst, und

(a) einen pH-Wert im Bereich von 3,5 bis 6,0 besitzt und
(b) - bezogen auf sein Gesamtgewicht - 0,4 bis 2,6 Gew.-% Calciumsuccinat enthält.

Unter geeigneten Haarbehandlungsmitteln sind bevorzugt Haarreinigungsmittel wie Shampoos, Haarpflegemittel wie Haarkuren, Spülungen oder Haarpflegesprays sowie Haarstylingmittel wie Haargele, Haarsprays oder Haarwachse zu verstehen. Ganz besonders bevorzugt ist das Haarbehandlungsmittel ein Shampoo.

Ein bevorzugter Gegenstand der vorliegenden Erfindung ist ein Shampoo zur Verringerung und/oder Vermeidung des Ausblutens und/oder Verblassens künstlich erzeugter Haarfarben, welches einen wässrigen kosmetischen Träger umfasst, und

(a) einen pH-Wert im Bereich von 3,5 bis 6,0 besitzt und
(b) - bezogen auf sein Gesamtgewicht - 0,4 bis 2,6 Gew.-% Calciumsuccinat enthält.

Unter künstlich erzeugten Haarfarben werden Färbungen verstanden, die durch Anwendung eines Haarfärbemittels auf Haaren erzeugt werden können. Hierbei enthalten diese Haarfärbemittel mindestens einen aus dem Stand der Technik bekannten Haarfarbstoff, insbesondere mindestens ein Oxidationsfarbstoffvorprodukt und/oder mindestens einen direktziehenden Farbstoff.

Das Ausbluten von künstlich erzeugten Haarfarben kann dann beobachtet werden, wenn gefärbte Haare entweder mit Wasser oder auch mit einer tensidhaltigen Lösung - wie beispielsweise einem Shampoo oder einer Haarkur - behandelt und anschließend gewaschen werden. Ein Ausbluten der Färbung macht sich dann in einer Färbung des Waschwassers bemerkbar. Das Ausbluten führt nicht nur zu einem Intensitätsverlust der gefärbten Haare, sondern bei Kontakt mit dem gefärbten Waschwasser auch zu einer Verschmutzung von Textilien.

Unter dem Verblassen der künstlich erzeugten Haarfarben ist ein Verlust in der Intensität der gefärbten Haare zu verstehen. Wie stark eine Haarfärbung verblasst, kann beispielsweise durch vergleichende farbmetrische Messungen vor und nach einer Haarwäsche ermittelt werden.

Das Haarbehandlungsmittel umfasst einen wässrigen kosmetischen Träger. Der kosmetische Träger kann beispielsweise wässrig oder auch wässrig-alkoholisch sein. Geeignete Träger sind beispielsweise Cremes, Emulsionen, Gele oder auch tensidhaltige schäumende Lösungen.

Unter wässrig-alkoholischen Lösungen sind im Sinne der vorliegenden Erfindung insbesondere wässrige Lösungen enthaltend 0,1 bis 70 Gew.-% eines $C_1$-$C_4$-Alkohols, insbesondere Ethanol bzw. Isopropanol, zu verstehen. Die Haarbehandlungsmittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

pH-Wert

Das Haarbehandlungsmittel besitzt (a) einen pH-Wert im Bereich von 3,5 bis 6,0. Im Zuge der zu dieser Erfindung führenden Arbeiten hat sich herausgestellt, dass der pH-Wert beim Farbschutz von gefärbten Haaren eine wesentliche Einflussgröße darstellt.

So wurde beobachtet, dass der Verlust der Farbintensität von gefärbten Haaren dann am geringsten ausfiel, wenn die Haare mit einem Mittel behandelt werden, dass auf einen pH-Wert im Bereich von 3,6 bis 4,9, bevorzugt von 3,8 bis 4,8, weiter bevorzugt von 4,0 bis 4,7 und ganz besonders bevorzugt von 4,2 bis 4,6 eingestellt wurde.

In den genannten bevorzugten und besonders bevorzugten pH-Werte-Bereichen konnte auch das Ausbluten der gefärbten Haarfasern bestmöglich unterdrückt werden.

In einer besonders bevorzugten Ausführungsform ist das Haarbehandlungsmittel dadurch gekennzeichnet, dass es (a) einen pH-Wert im Bereich von 3,6 bis 4,9, bevorzugt von 3,8 bis 4,8, weiter bevorzugt von 4,0 bis 4,7 und ganz besonders bevorzugt von 4,2 bis 4,6 besitzt.

Die Messung des pH-Wertes kann beispielsweise mit einer Glaselektrode erfolgen, die üblicherweise in Form einer Einstabmesskette kommerziell erwerblich ist. Vor der Messung des pH-Wertes werden die Glaselektroden üblicherweise mit Kalibrierlösungen bekannten pH-Wertes kalibriert. Unter den pH-Werten im Sinne der vorliegenden Erfindung werden pH-Werte verstanden, die bei einer Temperatur von 22 °C gemessen wurden.

Zur Einstellung des erfindungswesentlichen bzw. bevorzugten pH-Wertes können verschiedene Acidifizierungsmittel verwendet werden. Erfindungsgemäß bevorzugte Acidifizierungsmittel sind beispielsweise Zitronensäure, Milchsäure, Essigsäure oder auch verdünnte Mineralsäuren.

Obwohl die Haarbehandlungsmittel auf pH-Werte im sauren Bereich eingestellt werden, kann es dennoch nötig

sein, zur Feinjustierung des pH-Wertes in geringen Mengen auch Alkalisierungsmittel einzusetzen.

Erfindungsgemäß geeignete Alkalisierungsmittel können ausgewählt werden aus der Gruppe, die gebildet wird aus Ammoniak, Alkanolaminen, Alkalimetallhydroxiden, Alkalimetallmetasilikaten, Alkalimetallphosphaten und Alkalimetallhydrogenphosphaten. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin.

Erdalkalisalz einer Dicarbonsäure mit 2 bis 8 C-Atomen

Als erfindungswesentlichen Inhaltsstoff (b) enthalten die Haarbehandlungsmittel, die gemäß den erfindungsgemäßen Verwendungen verwendet werden, mindestens ein Erdalkalisalz einer Dicarbonsäure mit 2 bis 8 C-Atomen.

Erfindungsgemäße Dicarbonsäuren sind dadurch gekennzeichnet, dass sie 2 bis 8 Kohlenstoffatome und genau zwei Carbonsäuregruppierungen besitzen. Die beiden Carbonsäuregruppen liegen deprotoniert vor, und die beiden negativen Ladungen werden durch die zweifach positiv geladenen Erdalkali-Kationen (insbesondere $Mg^{2+}$ und $Ca^{2+}$) neutralisiert. Die Carbonsäuren können auch noch eine oder mehrere weitere Substituenten wie beispielsweise Hydroxygruppe(n), Oxogruppe(n) oder Aminogruppe(n) umfassen. Erfindungsgemäße Dicarbonsäuresalze können aliphatisch oder aromatisch, gesättigt oder ungesättigt sein.

Unter erfindungsgemäß besonders gut geeigneten Erdalkalisalzen von Dicarbonsäuren mit 2 bis 8 C-Atomen sind insbesondere die Magnesiumsalze und die Calciumsalze dieser Säuren zu verstehen.

In einer besonders bevorzugten Ausführungsform der beanspruchten Verwendungen ist ein Haarbehandlungsmittel dadurch gekennzeichnet, dass es (b) mindestens ein Magnesiumsalz und/oder ein Calciumsalz einer Dicarbonsäure mit 2 bis 8 C-Atomen enthält.

Der stärkste Farbschutz konnte beobachtet werden, wenn gefärbte Haare mit Mitteln behandelt wurden, die mindestens ein Calciumsalz einer Dicarbonsäure mit 2 bis 8 C-Atomen enthielten.

In einer ganz besonders bevorzugten Ausführungsform der beanspruchten Verwendungen ist ein Haarbehandlungsmittel dadurch gekennzeichnet, dass es (b) mindestens ein Calciumsalz einer Dicarbonsäure mit 2 bis 8 C-Atomen enthält.

Innerhalb der Gruppe der erfindungsgemäßen Erdalkalisalze von $C_2$-$C_8$-Dicarbonsäuren haben sich bestimmte Verbindungen als besonders vorteilhaft erwiesen. Aus diesem Grund ist es ganz besonders bevorzugt, wenn die erfindungsgemäßen Mittel mindestens ein Salz enthalten, das ausgewählt wird aus der Gruppe aus dem Calciumsalz der Bernsteinsäure (Succinsäure), dem Magnesiumsalz der Bernsteinsäure (Succinsäure), dem Calciumsalz der Malonsäure, dem Magnesiumsalz der Malonsäure, dem Calciumsalz der Maleinsäure, dem Magnesiumsalz der Maleinsäure, dem Calciumsalz der Fumarsäure, dem Magnesiumsalz der Fumarsäure, dem Calciumsalz der Glutaminsäure, dem Magnesiumsalz der Glutaminsäure, dem Calciumsalz der Asparaginsäure, dem Magnesiumsalz der Asparaginsäure, dem Calciumsalz der Oxalsäure, dem Magnesiumsalz der Oxalsäure, dem Calciumsalz der Glutarsäure, dem Magnesiumsalz der Glutarsäure, dem Calciumsalz der Adipinsäure, dem Magnesiumsalz der Adipinsäure, dem Calciumsalz der Pimelinsäure, dem Magnesiumsalz der Pimelinsäure, dem Calciumsalz der Suberinsäure, dem Magnesiumsalz der Suberinsäure, dem Calciumsalz der Tatronsäure, dem Magnesiumsalz der Tatronsäure, dem Calciumsalz der Weinsäure, dem Magnesiumsalz der Weinsäure, dem Calciumsalz der Äpfelsäure, dem Magnesiumsalz der Äpfelsäure, dem Calciumsalz der 2-Oxoglutarsäure, dem Magnesiumsalz der 2-Oxoglutarsäure, dem Calciumsalz der Oxobernsteinsäure und dem Magnesiumsalz der Oxobernsteinsäure.

Bersteinsäure trägt auch die Alternativnamen Succinsäure oder Butandisäure. Das Calciumsalz der Bernsteinsäure wird als Calciumsuccinat bezeichnet. Das Calciumsalz der Bernsteinsäure besitzt die Strukturformel $Ca^{2+}$ ($^-OOC$-$CH_2$-$CH_2$-$COO^-$) und hat die CAS-Nr. 140-99-8.

Das Magnesiumsalz der Bernsteinsäure wird als Magnesiumsuccinat bezeichnet. Das Magnesiumsalz der Bernsteinsäure besitzt die Strukturformel $Mg^{2+}$ ($^-OOC$-$CH_2$-$CH_2$-$COO^-$).

Bei Malonsäure handelt es sich um Propandisäure. Das Calciumsalz der Malonsäure besitzt die Strukturformel $Ca^{2+}$ ($^-OOC$-$CH_2$-$COO^-$). Das Magnesiumsalz der Malonsäure besitzt die Strukturformel $Mg^{2+}$ ($^-OOC$-$CH_2$-$COO^-$).

Maleinsäure wird auch als cis-Butendisäure bezeichnet. Das Calciumsalz der Maleinsäure besitzt die Strukturformel (I). Das Magnesiumsalz der Maleinsäure besitzt die Strukturformel (II).

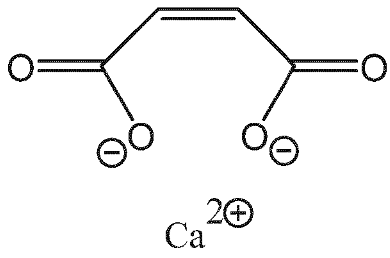
(I)

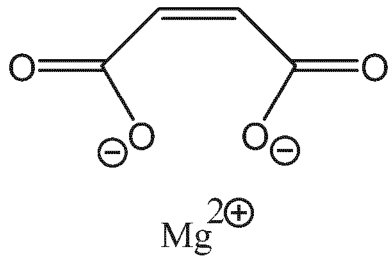
(II)

Fumarsäure trägt auch den Alternativnamen trans-Butendisäure. Das Calciumsalz der Fumarsäure besitzt die Strukturformel (III). Das Magnesiumsalz der Fumarsäure besitzt die Strukturformel (IV).

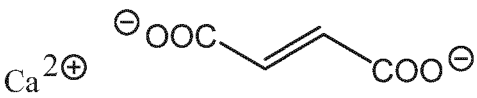
(III)

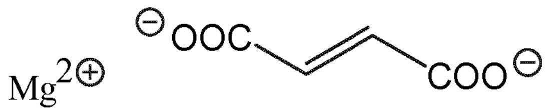
(IV)

Bei Glutaminsäure handelt es sich um die Aminosäure 2-Aminopentandisäure, die in Form von zwei Enantiomeren vorkommt. Sowohl die Erdalkali-Salze der (D)-Glutaminsäure, der (L)-Glutaminsäure wie auch ihrer Gemische sind erfindungsgemäß. Das Calciumsalz der Glutaminsäure besitzt die Strukturformel (V). Das Magnesiumsalz der Glutaminsäure besitzt die Strukturformel (VI).

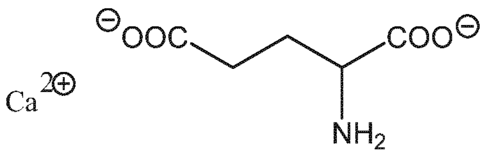
(V)

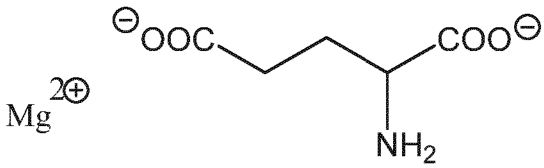
(VI)

Bei Asparaginsäure handelt es sich um die Aminosäure 2-Aminobutandisäure, die in Form von zwei Enantiomeren vorkommt. Sowohl die Erdalkali-Salze der (D)-Asparaginsäure, der (L)-Asparaginsäure als auch ihre Gemische sind erfindungsgemäß. Das Calciumsalz der Asparaginsäure besitzt die Strukturformel (VII). Das Magnesiumsalz der Asparaginsäure besitzt die Strukturformel (VIII).

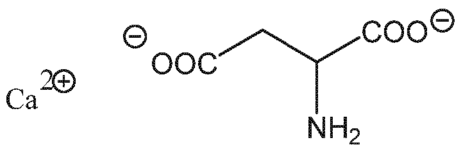
(VII)

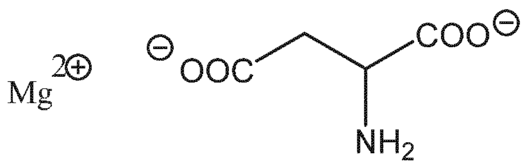
(VIII)

Oxalsäure wird auch als Ethandisäure bezeichnet. Das Calciumsalz der Oxalsäure trägt den Alternativnamen Calciumoxalat und besitzt die Strukturformel $Ca^{2+}(^{-}OOC\text{-}COO^{-})$. Das Magnesiumsalz der Oxalsäure besitzt die Strukturformel $Mg^{2+}(^{-}OOC\text{-}COO^{-})$.

Glutarsäure heißt alternativ auch Pentandisäure. Das Calciumsalz der Glutarsäure besitzt die Strukturformel $Ca^{2+}$ $(^{-}OOC\text{-}CH_2\text{-}CH_2\text{-}CH_2\text{-}COO^{-})$. Das Magnesiumsalz der Glutarsäure besitzt die Strukturformel $Mg^{2+}$ $(^{-}OOC\text{-}CH_2\text{-}CH_2\text{-}CH_2\text{-}COO^{-})$.

Adipinsäure wird auch als Hexandisäure bezeichnet. Das Calciumsalz der Adipinsäure besitzt die Strukturformel $Ca^{2+}$ $(^{-}OOC\text{-}CH_2\text{-}CH_2\text{-}CH_2\text{-}CH_2\text{-}COO^{-})$. Das Magnesiumsalz der Adipinsäure besitzt die Strukturformel $Mg^{2+}(^{-}OOC\text{-}CH_2\text{-}CH_2\text{-}CH_2\text{-}CH_2\text{-}COO^{-})$.

Ein Alternativname für Pimelinsäure ist auch Heptandisäure. Das Calciumsalz der Pimelinsäure besitzt die Strukturformel $Ca^{2+}$ $(^{-}OOC\text{-}CH_2\text{-}CH_2\text{-}CH_2\text{-}CH_2\text{-}CH_2\text{-}COO^{-})$. Das Magnesiumsalz der Pimelinsäure besitzt die Strukturformel $Mg^{2+}$ $(^{-}OOC\text{-}CH_2\text{-}CH_2\text{-}CH_2\text{-}CH_2\text{-}CH_2\text{-}COO^{-})$.

Bei Suberinsäure handelt es sich um Octandisäure. Das Calciumsalz der Suberinsäure besitzt die Strukturformel $Ca^{2+}$ $(^{-}OOC\text{-}CH_2\text{-}CH_2\text{-}CH_2\text{-}CH_2\text{-}CH_2\text{-}CH_2\text{-}COO^{-})$. Das Magnesiumsalz der Suberinsäure besitzt die Strukturformel $Mg^{2+}(^{-}OOC\text{-}CH_2\text{-}CH_2\text{-}CH_2\text{-}CH_2\text{-}CH_2\text{-}CH_2\text{-}COO^{-})$. Tatronsäure wird alternativ auch als 2-Hydroxypropandisäure bezeichnet. Das Calciumsalz der Tatronsäure besitzt die Strukturformel $Ca^{2+}(^{-}OOC\text{-}CH(OH)\text{-}COO^{-})$. Das Magnesiumsalz

der Tatronsäure besitzt die Strukturformel $Mg^{2+}(^{-}OOC\text{-}CH(OH)\text{-}COO^{-})$.

Weinsäure, alternativ auch 2,3-Dihydroxybernsteinsäure genannt, besitzt zwei optisch aktive Zentren und kommt sowohl in einer L-Form, einer D-Form als auch in einer meso-Form vor. Der Einsatz all dieser Formen wie auch ihrer Gemische ist erfindungsgemäß. Das Calciumsalz der Weinsäure wird auch Calciumtartrat genannt und besitzt die Strukturformel $Ca^{2+}(^{-}OOC\text{-}CH(OH)\text{-}CH(OH)\text{-}COO^{-})$. Das Magnesiumsalz der Weinsäure wird auch Magnesiumtartrat genannt und besitzt die Strukturformel $Mg^{2+}(^{-}OOC\text{-}CH(OH)\text{-}CH(OH)\text{-}COO^{-})$.

Äpfelsäure, alternativ auch 2-Hydroxybernsteinsäure genannt, besitzt ein optisch aktives Zentrum und kommt sowohl in einer L-Form als auch einer D-Form vor. Der Einsatz beider Formen wie auch ihres Gemisches ist erfindungsgemäß. Das Calciumsalz der Äpfelsäure besitzt die Strukturformel $Ca^{2+}(^{-}OOC\text{-}CH(OH)\text{-}CH_2\text{-}COO^{-})$. Das Magnesiumsalz der Äpfelsäure besitzt die Strukturformel $Mg^{2+}(^{-}OOC\text{-}CH(OH)\text{-}CH_2\text{-}COO^{-})$.

2-Oxoglutarsäure wird alternativ auch als 2-Oxopentandisäure bezeichnet. Das Calciumsalz der 2-Oxoglutarsäure besitzt die Strukturformel $(^{-}OOC\text{-}CH_2\text{-}CH_2\text{-}C(O)\text{-}COO^{-})$. Das Magnesiumsalz der Oxoglutarsäure besitzt die Strukturformel $Mg^{2+}(^{-}OOC\text{-}CH_2\text{-}CH_2\text{-}C(O)\text{-}COO^{-})$.

Ein Alternativname für Oxobernsteinsäure ist auch Oxobutandisäure. Das Calciumsalz der Oxobernsteinsäure besitzt die Strukturformel $Ca^{2+}$ $(^{-}OOC\text{-}CH_2\text{-}C(O)\text{-}COO^{-})$. Das Magnesiumsalz der Oxobernsteinsäure besitzt die Strukturformel $Mg^{2+}$ $(^{-}OOC\text{-}CH_2\text{-}C(O)\text{-}COO^{-})$.

In einer bevorzugten Ausführungsform ist das Haarbehandlungsmittel dadurch gekennzeichnet, dass es (b) mindestens ein Erdalkalisalz einer Dicarbonsäure mit 2 bis 8 C-Atomen enthält, das ausgewählt wird aus der Gruppe aus dem Calciumsalz der Bernsteinsäure (Succinsäure), dem Magnesiumsalz der Bernsteinsäure (Succinsäure), dem Calciumsalz der Malonsäure, dem Magnesiumsalz der Malonsäure, dem Calciumsalz der Maleinsäure, dem Magnesiumsalz der Maleinsäure, dem Calciumsalz der Fumarsäure, dem Magnesiumsalz der Fumarsäure, dem Calciumsalz der Glutaminsäure, dem Magnesiumsalz der Glutaminsäure, dem Calciumsalz der Asparaginsäure, dem Magnesiumsalz der Asparaginsäure, dem Calciumsalz der Oxalsäure, dem Magnesiumsalz der Oxalsäure, dem Calciumsalz der Glutarsäure, dem Magnesiumsalz der Glutarsäure, dem Calciumsalz der Adipinsäure, dem Magnesiumsalz der Adipinsäure, dem Calciumsalz der Pimelinsäure, dem Magnesiumsalz der Pimelinsäure, dem Calciumsalz der Suberinsäure, dem Magnesiumsalz der Suberinsäure, dem Calciumsalz der Tatronsäure, dem Magnesiumsalz der Tatronsäure, dem Calciumsalz der Weinsäure, dem Magnesiumsalz der Weinsäure, dem Calciumsalz der Äpfelsäure, dem Magnesiumsalz der Äpfelsäure, dem Calciumsalz der 2-Oxoglutarsäure, dem Magnesiumsalz der 2-Oxoglutarsäure, dem Calciumsalz der Oxobernsteinsäure und dem Magnesiumsalz der Oxobernsteinsäure.

In einer besonders bevorzugten Ausführungsform ist das Haarbehandlungsmittel dadurch gekennzeichnet, dass es (b) mindestens ein Erdalkalisalz einer Dicarbonsäure mit 2 bis 8 C-Atomen enthält, das ausgewählt wird aus der Gruppe aus dem Calciumsalz der Bernsteinsäure (Succinsäure), dem Magnesiumsalz der Bernsteinsäure (Succinsäure), dem Calciumsalz der Malonsäure, dem Magnesiumsalz der Malonsäure, dem Calciumsalz der Maleinsäure, dem Magnesiumsalz der Maleinsäure, dem Calciumsalz der Fumarsäure, dem Magnesiumsalz der Fumarsäure, dem Calciumsalz der Glutaminsäure, dem Magnesiumsalz der Glutaminsäure, dem Calciumsalz der Asparaginsäure, dem Magnesiumsalz der Asparaginsäure, dem Calciumsalz der Glutarsäure, dem Magnesiumsalz der Glutarsäure, dem Calciumsalz der Adipinsäure, dem Magnesiumsalz der Adipinsäure, dem Calciumsalz der Pimelinsäure, dem Magnesiumsalz der Pimelinsäure, dem Calciumsalz der Suberinsäure, dem Magnesiumsalz der Suberinsäure, dem Calciumsalz der Tatronsäure, dem Magnesiumsalz der Tatronsäure, dem Calciumsalz der Weinsäure, dem Magnesiumsalz der Weinsäure, dem Calciumsalz der Äpfelsäure, dem Magnesiumsalz der Äpfelsäure, dem Calciumsalz der 2-Oxoglutarsäure, dem Magnesiumsalz der 2-Oxoglutarsäure, dem Calciumsalz der Oxobernsteinsäure und dem Magnesiumsalz der Oxobernsteinsäure.

In einer ganz besonders bevorzugten Ausführungsform ist das Haarbehandlungsmittel dadurch gekennzeichnet, dass es (b) mindestens ein Erdalkalisalz einer Dicarbonsäure mit 2 bis 8 C-Atomen enthält, das ausgewählt wird aus der Gruppe aus dem Calciumsalz der Bernsteinsäure (Succinsäure), dem Calciumsalz der Malonsäure, dem Calciumsalz der Maleinsäure, dem Calciumsalz der Fumarsäure, dem Calciumsalz der Glutaminsäure, dem Calciumsalz der Asparaginsäure, dem Calciumsalz der Glutarsäure, dem Calciumsalz der Adipinsäure, dem Calciumsalz der Pimelinsäure, dem Calciumsalz der Suberinsäure, dem Calciumsalz der Tatronsäure, dem Calciumsalz der Weinsäure, dem Calciumsalz der Äpfelsäure, dem Calciumsalz der 2-Oxoglutarsäure, dem Calciumsalz der Oxobernsteinsäure und dem Magnesiumsalz der Oxobernsteinsäure.

Innerhalb der Gruppe der vorgenannten Salze am allermeisten bevorzugt ist das Calciumsalz der Bernsteinsäure (Calciumsuccinat).

In einer ganz besonders bevorzugten Ausführungsform ist das Haarbehandlungsmittel dadurch gekennzeichnet, dass es (b) das Calciumsalz der Bernsteinsäure (Calciumsuccinat) enthält.

Es hat sich herausgestellt, dass der beste Farbschutz erzielt werden kann, wenn das Mittel die Erdalkalisalze der Säure(n) in bestimmten Mengenbereichen enthält. In diesem Zusammenhang hat es sich als besonders vorteilhaft herausgestellt, ein oder mehrere Erdalkalisalze von Dicarbonsäuren mit 2 bis 8 C-Atomen in einer Gesamtmenge von 0,1 bis 3,5 Gew.-%, bevorzugt von 0,3 bis 2,8 Gew.-%, weiter bevorzugt von 0,6 bis 2,4 Gew.-% und ganz besonders

bevorzugt von 0,8 bis 1,6 Gew.-% im Mittel einzusetzen. Alle Angaben in Gew.-% sind hierbei auf das Gesamtgewicht der im Mittel enthaltenen Erdalkalisalze der Säuren verstanden, das zum Gesamtgewicht des Mittels in Relation gesetzt wird.

In einer weiteren besonders bevorzugten Ausführungsform ist das Haarbehandlungsmittel dadurch gekennzeichnet, dass es - bezogen auf sein Gesamtgewicht - (b) ein oder mehrere Erdalkalisalze von Dicarbonsäuren mit 2 bis 8 C-Atomen in einer Gesamtmenge von 0,1 bis 3,5 Gew.-%, bevorzugt von 0,3 bis 2,8 Gew.-%, weiter bevorzugt von 0,6 bis 2,4 Gew.-% und ganz besonders bevorzugt von 0,8 bis 1,6 Gew.-% enthält.

Wie zuvor beschrieben ist der Einsatz von Calciumsuccinat im Haarbehandlungsmittel ganz besonders bevorzugt, da mit Calciumsuccinat die allerbesten Effekte erzielt werden konnten.

In einer explizit ganz besonders bevorzugten Ausführungsform ist das Haarbehandlungsmittel dadurch gekennzeichnet, dass es - bezogen auf sein Gesamtgewicht - (b) 0,4 bis 2,6 Gew.-%, bevorzugt 0,7 bis 2,3 Gew.-%, weiter bevorzugt 0,9 bis 1,5 Gew.-% und ganz besonders bevorzugt 1,2 bis 1,4 Gew.-% Calciumsuccinat enthält.

Anionische Tenside

Bei der Vermessung der Waschechtheiten von gefärbten Haarsträhnen wurde beobachtet, dass sich mit den Erdalkalisalzen von C2-C8-Dicarbonsäuren im Vergleich zu anderen Salzen (wie beispielsweise den Alkalisalzen dieser Säuren) eine verbesserte Waschechtheit und ein vermindertes Ausbluten der Färbungen erzielen lässt.

Als problematisch hat sich jedoch herausgestellt, dass die Löslichkeit dieser Erdalkalisalze geringer ist als die Löslichkeit der entsprechenden Alkalisalze. Optimale Ergebnisse konnten daher insbesondere bei bestmöglicher Solubilisation der Erdalkalisalze erzielt werden.

Eine ausreichende Löslichkeit konnte zum einen durch Wahl der optimalen Einsatzmengen und pH-Werte erzielt werden. Darüber hinaus hat sich jedoch ebenfalls herausgestellt, dass das Hinzufügen von mindestens einem Aniontensid zu den Mitteln die Salze bestmöglich löst bzw. feinteilig dispergiert. Aus diesem Grund ist es ganz besonders bevorzugt, den erfindungsgemäßen Mitteln zusätzlich mindestens ein anionisches Tensid hinzufügen. In diesem Zusammenhang als besonders gut geeignet haben sich die Alkylsulfate, Alkylethersulfate, Sulfosuccinate und Ethercarbonsäuren erwiesen.

In einer weiteren besonders bevorzugten Ausführungsform ist ein erfindungsgemäßes Haarbehandlungsmittel dadurch gekennzeichnet, dass es mindestens ein anionisches Tensid aus der Gruppe aus Alkylsulfaten, Alkylethersulfaten, Ethercarbonsäuren und Sulfosuccinaten enthält.

Alkylsulfat- und/oder Alkylethersulfatsalze besitzen die allgemeine Formel $R\text{-}(OCH_2\text{-}CH_2)_x\text{-}OSO_3^{-}M^{+}$, in der R bevorzugt eine lineare oder verzweigte, gesättigte oder ungesättigte Alkylgruppe mit 8 bis 30 C-Atomen ist, x die Zahl 0 oder 1 bis 12 darstellt und M ein Alkali-, Ammonium- oder Alkanolaminion bedeutet.

Besonders bevorzugte anionische Tenside sind geradkettige oder verzweigte Alkylulfate der zuvor genannten Formel, die einen Alkylrest mit 8 bis 18, insbesondere mit 10 bis 16 C-Atomen enthalten. x steht in diesem Fall für die Zahl 0. Insbesondere bevorzugt sind die Natrium-, Kalium- und/oder Triethanolaminsalze linearer oder verzweigter Lauryl-, Tridecyl- und/oder Myristylsulfate.

Besonders bevorzugte anionische Tenside sind geradkettige oder verzweigte Alkylethersulfate der zuvor genannten Formel, die einen Alkylrest mit 8 bis 18, insbesondere mit 10 bis 16 C-Atomen, sowie 1 bis 6 und insbesondere 2 bis 4 Ethylenoxideinheiten enthalten (d.h. x steht für eine ganze Zahl von 1 bis 6, insbesondere für eine ganze Zahl von 2 bis 4). Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Bevorzugte Ethercarbonsäuren besitzen die allgemeine Formel $R\text{-}O\text{-}(CH_2\text{-}CH_2O)_x\text{-}CH_2\text{-}COOM$, in der R eine lineare oder verzweigte, gesättigte oder ungesättigte Alkylgruppe mit 8 bis 30 C-Atomen und x = 0 oder 1 bis 16 ist. M steht für ein Wasserstoffatom oder für ein Alkali-, Ammonium- oder Alkanolaminion.

Wird das Haarbehandlungsmittel als Haarshampoo konfektioniert, enthält es bevorzugt mindestens ein anionisches Tensid in einem bevorzugten Gewichtsanteil von 0,5 bis 20 Gew.-%, mehr bevorzugt von 1 bis 15 und insbesondere bevorzugt von 2 bis 12 Gew.-%, wobei sich die Mengenangaben auf das Gesamtgewicht des Haarbehandlungsmittels beziehen.

Besonders gut lassen sich die Erdalkalisalze von C2-C8-Dicarbonsäuren durch Alkylethersulfate solubilisieren bzw. dispergieren. Daher ist es besonders bevorzugt, wenn die Haarbehandlungsmittel mindestens ein Alkylethersulfat (beispielsweise in Form des Natriumsalzes) enthalten, das - bezogen auf das Gesamtgewicht des Mittels - in Mengen von 5,5 bis 16,0 Gew.-%, bevorzugt von 6,5 bis 14,0 Gew.-%, weiter bevorzugt von 7,5 bis 12,0 Gew.-% und ganz besonders bevorzugt von 8,0 bis 10,0 Gew.-% - eingesetzt wird.

In einer weiteren besonders bevorzugten Ausführungsform ist das Haarbehandlungsmittel dadurch gekennzeichnet, dass es - bezogen auf sein Gesamtgewicht - ein oder mehrere anionische Tenside aus der Gruppe der Alkylethersulfate in einer Gesamtmenge von 5,5 bis 16,0 Gew.-%, bevorzugt von 6,5 bis 14,0 Gew.-%, weiter bevorzugt von 7,5 bis 12,0 Gew.-% und ganz besonders bevorzugt von 8,0 bis 10,0 Gew.-% enthält.

Amphotere und/oder zwitterionische Tenside

Weiterhin hat es sich als besonders bevorzugt herausgestellt, wenn das Mittel Mindestens ein amphoteres und/oder zwitterionisches Tensid enthalten.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Besonders gut kompatibel mit den Erdalkalisalzen von $C_2$-$C_8$-Dicarbonsäuren sind die Betaine, insbesondere die N-Alkyl-N,N-dimethylammonium-glycinaten und/oder die N-Acyl-aminopropyl-N,N-dimethylammoniumglycinate.

Unter amphoteren Tensiden werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine. Besonders bevorzugte ampholytische Tenside sind das N-Kokosalkylaminopropionat, das Kokosacylaminoethylaminopropionat und das $C_{12}$ - $C_{18}$ - Acylsarcosin. Besonders gut kompatibel mit den Erdalkalisalzen von $C_2$-$C_8$-Dicarbonsäuren sind die 2-Alkyl-3-carboxymethyl-3-hydroxyethyl-imidazoline, die N-Alkylglycine, die N-Alkylpropionsäure, die N-Alkylaminobuttersäuren, die N-Alkyliminodipropionsäuren, die N-Hydroxyethyl-N-alkylamidopropylglycinen, die N-Alkyltaurine, die N-Alkylsarcosine, die 2-Alkylaminopropionsäuren, den Alkylaminoessigsäuren, die N-Alkylamphodiacetate und/oder die N-Alkylamphodipropionate.

In einer weiteren besonders bevorzugten Ausführungsform ist das Haarbehandlungsmittel dadurch gekennzeichnet, dass es mindestens ein amphoteres und/oder zwitterionisches Tensid aus der Gruppe der Betaine, der 2-Alkyl-3-carboxymethyl-3-hydroxyethyl-imidazoline, der N-Alkylglycine, der N-Alkylpropionsäuren, der N-Alkylaminobuttersäuren, der N-Alkyliminodipropionsäuren, der N-Hydroxyethyl-N-alkylamidopropylglycine, der N-Alkyltaurine, der N-Alkylsarcosine, der 2-Alkylaminopropionsäuren und der Alkylaminoessigsäuren, der N-Alkylamphodiacetate und/oder der N-Alkylamphodipropionate enthält.

Auch die amphoteren und/oder zwitterionischen Tenside werden bevorzugt in bestimmten Mengen im erfindungsgemäßen Mittel eingesetzt, die - bezogen auf das Gesamtgewicht des Mittels - im Bereich von 1,5 bis 6,5 Gew.-%, bevorzugt von 2,5 bis 5,5 Gew.-%, und besonders bevorzugt von 3,5 bis 4,5 Gew.-% liegen können.

In einer weiteren bevorzugten Ausführungsform ist das Haarbehandlungsmittel dadurch gekennzeichnet, dass es - bezogen auf das Gesamtgewicht des Mittels - ein oder mehrere zwitterionische und/oder amphotere Tenside in einer Gesamtmenge von 1,5 bis 6,5 Gew.-%, bevorzugt von 2,5 bis 5,5 Gew.-%, und besonders bevorzugt von 3,5 bis 4,5 Gew.-% enthält.

Explizit ganz besonders bevorzugt ist der Einsatz von Betainen, insbesondere von Cocoamidopropylbetain.

In einer ganz besonders bevorzugten Ausführungsform ist das Haarbehandlungsmittel dadurch gekennzeichnet, dass es - bezogen auf sein Gesamtgewicht - ein oder mehrere zwitterionische Tenside aus der Gruppe der Betaine in einer Gesamtmenge von 1,5 bis 6,5 Gew.-%, bevorzugt von 2,5 bis 5,5 Gew.-%, und besonders bevorzugt von 3,5 bis 4,5 Gew.-% enthält.

In einer ganz besonders bevorzugten Ausführungsform ist das Haarbehandlungsmittel dadurch gekennzeichnet, dass es - bezogen auf sein Gesamtgewicht -1,5 bis 6,5 Gew.-%, bevorzugt von 2,5 bis 5,5 Gew.-%, und besonders bevorzugt von 3,5 bis 4,5 Gew.-% Cocoamidopropylbetain enthält.

Weitere Inhaltsstoffe

Die Haarbehandlungsmittel können zusätzlich weitere Wirk-, Hilfs- und Zusatzstoffe enthalten, wie beispielsweise kationische Tenside, nichtionische Tenside, anionische, nichtionische und/oder kationische Polymere, Strukturanten wie Glucose, Parfümöle, faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Farbverändernde Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Vitamine, Provitamine und Vitaminvorstufen; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, $N_2O$, Dimethylether, $CO_2$ und Luft.

Die zusätzlichen Wirk- und Hilfsstoffe werden in den Haarbehandlungsmitteln bevorzugt in Mengen von jeweils 0,0001 bis 10 Gew.-%, insbesondere von 0,0005 bis 5 Gew.-%, bezogen auf das Gesamtgewicht des Mittels (A) bzw. der Oxidationsmittelzubereitung (B) eingesetzt.

<u>Verfahren zur Verringerung und/oder Vermeidung des Ausblutens und/oder Verblassens künstlich erzeugter Haarfarben</u>

Einsatzzweck der zuvor beschriebenen Haarbehandlungsmittel des ersten Erfindungsgegenstands sind Verfahren zur Verringerung und/oder Vermeidung des Ausblutens und/oder Verblassens künstlich erzeugter Haarfarben. Mit anderen Worten werden die erfindungsgemäßen Mittel zum Farbschutz von künstlich gefärbten Haaren eingesetzt. Wenn die erfindungsgemäßen Mittel auf Haare appliziert werden, die zuvor mit Oxidationsfarbstoffen und/oder direktziehenden Farbstoffen gefärbt worden waren, so wurde das Verblassen, Auswaschen und Ausbluten dieser Haare effektiv minimiert.

Ein zweiter Gegenstand der vorliegenden Erfindung ist daher ein Verfahren zur Verringerung und/oder Vermeidung des Ausblutens und/oder Verblassens künstlich erzeugter Haarfarben, umfassend die folgenden Schritte in der angegebenen Reihenfolge

(I) Applizieren eines Färbemittels auf Haare, wobei das Färbemittel mindestens ein Oxidationsfarbstoffvorprodukt und/oder mindestens einen direktziehenden Farbstoff enthält
(II) Einwirkenlassen des Färbemittels auf die Haare für einen Zeitraum von 5 Minuten bis 45 Minuten
(III) Ausspülen des Färbemittels aus den Haaren
(IV) Applizieren eines Farbschutzmittels auf die Haare, wobei es sich bei dem Farbschutzmittel um ein Haarbehandlungsmittel handelt, wie es bei der Beschreibung des ersten Erfindungsgegenstands im Detail offenbart wurde,
(V) Einwirkenlassen des Farbschutzmittels für einen Zeitraum von 5 Minuten bis 45 Minuten.

In Schritt (I) des erfindungsgemäßen Verfahrens wird zunächst ein Färbemittel auf die Haare appliziert, welches mindestens ein Oxidationsfarbstoffvorprodukt und/oder mindestens einen direktziehenden Farbstoff enthält.

Oxidative Färbungen werden mit Oxidationsfarbstoffvorprodukten auf Basis von Entwickler- und Kupplerkomonenten auf den Keratinfasern erzeugt. Oxidationsfarbstoffvorprodukte vom Entwicklertyp sind beispielsweise p-Phenylendiamin, p-ToluylendiaminN,N-Bis-(β-hydroxyethyl)-p-phenylendiamin, 4-N,N-Bis-(β-hydroxyethyl)-amino-2-methylanilin, 2-(β-Hydroxyethyl)-p-phenylendiamin, 2-(α,β-Dihydroxyethyl)-p-phenylendiamin, 2-Hydroxymethyl-p-phenylendiamin, Bis-(2-hydroxy-5-aminophenyl)-methan, p-Aminophenol, 4-Amino-3-methylphenol, 2,4,5,6-Tetraaminopyrimidin, 4-Hydroxy-2,5,6-triaminopyrimidin, 2-Hydroxy-4,5,6-triaminopyrimidin und/oder 4,5-Diamino-1-(β-hydroxyethyl)-pyrazol.

Oxidationsfarbstoffvorprodukte vom Kupplertyp sind beispielsweise m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol, 1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die in Schritt (I) des Verfahrens eingesetzten Färbemittel können auch einen oder mehrere direktziehende Farbstoffe enthalten. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Weiterhin können die zu entfärbenden Substrate auch mit in der Natur vorkommenden, natürlichen direktziehenden Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Ein ganz besonders bevorzugtes Verfahren ist gekennzeichnet durch das (I) Applizieren eines oxidativen Färbmittels auf Haaren, wobei das oxidative Färbemittel mindestens ein Oxidationsfarbstoffvorprodukt enthält.

In einem oxidativen Färbemittel werden die Färbungen unter Einfluss eines Oxidationsmittels (insbesondere Wasserstoffperoxid) aus den Oxidationsfarbstoffvorprodukten erzeugt. Bei dem in Schritt (I) des Verfahrens applizierten oxidativen Färbemittel handelt es sich daher um ein anwendungsbereites Färbemittel, das neben dem bzw. den Oxidationfarbstoffvorprodukten auch mindestens ein Oxidationsmittel (insbesondere Wasserstoffperoxid) enthält.

Ein ganz besonders bevorzugtes Verfahren ist deshalb gekennzeichnet durch das (I) Applizieren eines oxidativen Färbmittels auf Haare, wobei das oxidative Färbemittel mindestens ein Oxidationsfarbstoffvorprodukt vom Entwicklertyp, mindestens ein Oxidationsfarbstoffvorprodukt vom Kupplertyp und Wasserstoffperoxid enthält.

Die in Schritt (I) auf die Haare applizierten Färbemittel werden dann in Schritt (II) für einen Zeitraum von 5 bis 45 Minuten einwirken gelassen und im Anschluss daran in Schritt (III) ausgespült.

Die Schritte (I), (II) und (III) des Verfahrens sind Teile des Färbeverfahrens und werden daher zeitlich unmittelbar hintereinander, d.h. innerhalb eines Zeitraums von wenigen Stunden, durchgeführt.

Nach dem Ausspülen des Färbemittels (Schritt III) können die Haare optional getrocknet werden. Es ist jedoch ebenfalls möglich, das Farbschutzmittel (Schritt (IV)) auf die noch feuchten Haare zu applizieren. Bei dem in Schritt (IV) verwendeten Farbschutzmittel handelt es sich um ein Mittel des ersten Erfindungsgegenstands.

Zwischen der Durchführung der Schritte (III) und (IV) kann ein Zeitraum von einigen Stunden bis zu mehreren Tagen liegen.

Bevorzugt werden die Schritte (III) und (IV) innerhalb eines Zeitraumes von 1 bis 72 Stunden, bevorzugt von 1 bis 48 Stunden, ganz besonders bevorzugt von 1 bis 6 Stunden, durchgeführt.

Ganz besonders bevorzugt ist ein Verfahren zur Verringerung und/oder Vermeidung des Ausblutens und/oder Verblassens künstlich erzeugter Haarfarben, umfassend die folgenden Schritte in der angegebenen Reihenfolge

(I) Applizieren eines oxidativen Färbmittels auf Haare, wobei das oxidative Färbemittel mindestens ein Oxidationsfarbstoffvorprodukt vom Entwicklertyp, mindestens ein Oxidationsfarbstoffvorprodukt vom Kupplertyp und Wasserstoffperoxid enthält,

(II) Einwirkenlassen des Färbemittels auf die Haare für einen Zeitraum von 5 Minuten bis 45 Minuten

(III) Ausspülen des Färbemittels aus den Haaren

(IV) Applizieren eines Farbschutzmittels auf die Haare, wobei es sich bei dem Farbschutzmittel um ein Haarbehandlungsmittel handelt, wie es bei der Beschreibung des ersten Erfindungsgegenstands im Detail offenbart wurde,

(V) Einwirkenlassen des Farbschutzmittels für einen Zeitraum von 5 Minuten bis 45 Minuten, wobei die Schritte (III) und (IV) innerhalbe eines Zeitraums von 1 bis 72 Stunden, bevorzugt von 1 bis 24 Stunden, ganz besonders bevorzugt von 1 bis 6 Stunden, durchgeführt werden.

Nach dem Applizieren des Farbschutzmittels wird dieses in Schritt (V) für einen Zeitraum von 5 Minuten bis 45 Minuten einwirken gelassen.

Das Farbschutzmittel, d.h. das erfindungsgemäße Haarbehandlungsmittel des ersten Erfindungsgegenstands, kann als "rinse-off" oder als "leave-on" Produkt konzipiert werden.

Wie bereits zuvor beschrieben ist jedoch ganz besonders bevorzugt, wenn das auf die entsprechenden pH-Werte eingestellte Mittel neben Wasser und den Erdalkalisalzen von $C_2$-$C_8$-Dicarbonsäuren zusätzlich auch noch die zuvor offenbarten anionischen und/oder amphoteren-/zwitterionischen Tenside enthält. Enthält das Mittel diese Tenside in höheren Mengen, ist die Konfektionierung als "rinse-off" Produkt bevorzugt.

In einer weiteren ganz besonders bevorzugten Ausführungsform ist ein Verfahren dadurch gekennzeichnet, dass Schritt (V) gefolgt ist vom

(VI) Ausspülen des Farbschutzmittels aus den Haaren.

Ganz besonders bevorzugt ist ein Verfahren zur Verringerung und/oder Vermeidung des Ausblutens und/oder Verblassens künstlich erzeugter Haarfarben, umfassend die folgenden Schritte in der angegebenen Reihenfolge

(I) Applizieren eines oxidativen Färbmittels auf Haare, wobei das oxidative Färbemittel mindestens ein Oxidationsfarbstoffvorprodukt vom Entwicklertyp, mindestens ein Oxidationsfarbstoffvorprodukt vom Kupplertyp und Wasserstoffperoxid enthält,
(II) Einwirkenlassen des Färbemittels auf die Haare für einen Zeitraum von 5 Minuten bis 45 Minuten
(III) Ausspülen des Färbemittels aus den Haaren
(IV) Applizieren eines Farbschutzmittels auf die Haare, wobei es sich bei dem Farbschutzmittel um ein Haarbehandlungsmittel handelt, wie es bei der Beschreibung des ersten Erfindungsgegenstand im Detail offenbar wurde,
(V) Einwirkenlassen des Farbschutzmittels für einen Zeitraum von 5 Minuten bis 45 Minuten, und
(VI) Ausspülen des Farbschutzmittels aus den Haaren,

wobei die Schritte (III) und (IV) innerhalb eines Zeitraums von 1 bis 72 Stunden, bevorzugt 1 bis 24 Stunden, ganz besonders bevorzugt 1 bis 6 Stunden, durchgeführt werden.

Betreffend die bevorzugten Ausführungsformen des erfindungsgemäßen Verfahrens gilt mutatis mutandis das zum erfindungsgemäßen Haarbehandlungsmittel gesagte.

Mehrkomponenten-Verpackungseinheit (Kit-of-Parts)

Für den Anwender ist es zur Durchführung des zuvor beschriebenen Verfahrens besonders komfortabel, wenn ihm alle hierfür notwendigen Mittel in Form einer Mehrkomponenten-Verpackungseinheit (Kit-of-Parts) zur Verfügung gestellt werden.

Ein dritter Gegenstand der vorliegenden Erfindung ist daher eine Mehrkomponenten-Verpackungseinheit (Kit-of-Parts) zum Behandeln von keratinischen Fasern, insbesondere menschlichen Haaren, umfassend getrennt voneinander konfektioniert

- einen Container (I) enthaltend ein kosmetisches Mittel (A) und
- einen Container (II) enthaltend ein kosmetisches Mittel (B),

wobei

- das Mittel (A) in Container (I) ein Färbemittel ist, das mindestens ein Oxidationsfarbstoffvorprodukt und/oder mindestens einen direktziehenden Farbstoff enthält und
- das Mittel (B) in Container (II) ein Haarbehandlungsmittel ist, wie es bei der Beschreibung des ersten Erfindungsgegenstands im Detail offenbart wurde.

Im Fall von oxidativen Färbmitteln wird auf die Haare üblicherweise ein anwendungsbereites Färbemittel appliziert, das kurz vor der Anwendung durch Vermischen von einer Farbcreme (enthaltend das bzw. die Oxidationsfarbstoffvorprodukte) und einer Oxidationsmittelzubereitung (enthaltend als Oxidationsmittel Wasserstoffperoxd) erhalten wurde.

In diesem Zusammenhang ganz besonders bevorzugt ist eine Mehrkomponenten-Verpackungseinheit (Kit-of-Parts) zum Behandeln von keratinischen Fasern, insbesondere menschlichen Haaren, umfassend getrennt voneinander konfektioniert

- einen Container (I) enthaltend ein kosmetisches Mittel (A1) und
- einen Container (II) enthaltend ein kosmetisches Mitetl (A2) und
- einen Container (III) enthaltend ein kosmetisches Mittel (B),

wobei

- das Mittel (A1) in Container (I) ein Färbemittel ist, das mindestens ein Oxidationsfarbstoffvorprodukt und/oder mindestens einen direktziehenden Farbstoff enthält und
- das Mittel (A2) in Container (II) eine Oxidationsmittelzubereitung ist, die Wasserstoffperoxid enthält und
- das Mittel (B) in Container (III) ein Haarbehandlungsmittel ist, wie es bei der Beschreibung des ersten Erfindungsgegenstands im Detail offenbart wurde.

Weiterhin ist es nicht ausgeschlossen, wenn die erfindungegemäße Mehrkomponenten-Verpackungseinheit - neben den Containern (I), (II) sowie gegebenenfalls (III) - auch noch einen weiteren Container (IV) enthält. Dieser Container (IV) kann beispielsweise eine zweite Portion des Farbschutzmittels oder auch ein anderes Mittel (z.B. einen Conditioner) enthalten.

Betreffend die bevorzugten Ausführungsformen der erfindungsgemäßen Mehrkomponenten-Verpackungseinheit gilt mutatis mutandis das zum erfindungsgemäßen Haarbehandlungsmittel und Verfahren gesagte.

Ein weiterer Gegenstand der Erfindung ist die Verwendung eines , das einen wässrigen kosmetischen Träger umfasst, und(a) einen pH-Wert im Bereich von 3,5 bis 6,0 besitzt und(b) mindestens ein Erdalkalisalz einer Dicarbonsäure mit 2 bis 8 C-Atomen enthält, zum Farbschutz von gefärbten Haaren.

Ein weiterer Gegenstand der Erfindung ist die Verwendung dieses Haarbehandlungsmittels zur Verringerung und/oder Vermeidung des Ausblutens und/oder Verblassens künstlich erzeugter Haarfarben.

Betreffend die bevorzugten Ausführungsformen der erfindungsgemäßen Verwendung gilt mutatis mutandis das, was zuvor beschrieben wurde.

Beispiele:

1. Färbung von Haarsträhnen

Es wurden die folgenden Färbemittel hergestellt (alle Angaben in Gew.-%)

| | FC1 | FC2 |
|---|---|---|
| Cetearylalkohol | 12,0 | 12,0 |
| Ammoniumhydroxid | 4,7 | 4,7 |
| Glycerylmonostearat | 3,4 | 3,4 |
| Ceteareth-20 | 2,9 | 2,9 |
| 2-Octyldodecanol | 2,0 | 2,0 |
| Natriumlaureth-Sulfate (C12-C14, 2 - 3 EO) | 1,2 | 1,2 |
| Glycerin | 1,0 | 1,0 |
| Natriumcetearylsulfat | 0,7 | 0,7 |
| Ölsäure | 0,4 | 0,4 |
| Natriumsulfit | 0,4 | 0,2 |
| Kaliumstearat | 0,3 | 0,3 |
| Ethanolamin | 0,2 | 0,4 |
| EDTA, Tetranatriumsalz | 0,2 | 0,2 |
| Carbomer (Polyacrylsäure, Homopolymer) | 0,15 | 0,15 |
| Polyquaternium-39 | 0,15 | 0,15 |
| Kaliumhydroxid | 0,05 | 0,05 |
| Ascorbinsure | 0,05 | 0,05 |
| Linoleamidopropyl PG-Dimonium Chloride Phosphate | 0,03 | 0,03 |
| Titandioxid | 0,5 | 0,5 |
| 1-Hydroxyethyl 4,5-Diamino Pyrazole Sulfate | 1,90 | 0,94 |
| 4-Amino-2-hydroxytoluene | 0,80 | --- |
| Toluene-2,5-diamin, Sulfat | 0,35 | 0,04 |
| m-Aminophenol | 0,30 | 0,06 |
| 1-Naphthol | --- | 0,33 |
| 6-Methoxy-2-methylamino-3-aminopyridin, HCl | --- | 0,21 |
| 2-Amino-4-[(2-hydroxyethyl)amino]-anisol, Sulfat | --- | 0,08 |
| Wasser | ad 100 | ad 100 |

Die Farbcremes FC1 und FC2 wurden jeweils im Verhältnis 1:1 mit einer 6 %igen wässrigen Wasserstoffperoxidlösung (OX) vermischt. Die auf diese Weise hergestellten anwendungsbereiten Färbemittel wurde auf geschädigte

Haarsträhnen (Kerling Euronaturhaar 7/0, 1 x ultrablondiert) aufgetragen, für 30 Minuten dort belassen und anschließend mit Wasser ausgespült. Nach dem Trocknen wurden die gefärbten Haarsträhnen farbmetrisch vermessen (Messung der Lab-Werte, Datacolor Spectraflash SF 450, Lichtart: D65, Programm: Datacolor Tools 2.0.1).

2. Farbschutz Shampoos

Es wurden die folgenden Shampoos hergestellt (alle Angaben in Gew.-%)

| | Shampoo V1 | Shampoo V2 | Shampoo V3 | Shampoo E1 |
|---|---|---|---|---|
| Natriumlaureth-Sulfate (C12-C14, 2 - 3 EO) | 8,8 | 8,8 | 8,8 | 8,8 |
| Natiumhydroxid | 0,05 | 0,05 | 0,05 | 0,05 |
| Benzophenon-4 | 0,1 | 0,1 | 0,1 | 0,1 |
| Euperlan PK 3000 AM (1) | 2,0 | 2,0 | 2,0 | 2,0 |
| Natriumbenzoat | 0,5 | 0,5 | 0,5 | 0,5 |
| D-Panthenol | 0,2 | 0,2 | 0,2 | 0,2 |
| Croquat WKP PE LQ (1) | 0,05 | 0,05 | 0,05 | 0,05 |
| Dinatriumcocoamphodiacetat | 2,0 | 2,0 | 2,0 | 2,0 |
| Coconut fatty acid monoethanolamide | 0,7 | 0,7 | 0,7 | 0,7 |
| PEG-7 Glycerylcocoat | 0,85 | 0,85 | 0,85 | 0,85 |
| Hydrogenated Castor Oil | 0,25 | 0,25 | 0,25 | 0,25 |
| PEG-40 hydrogenated Castor Oil | 0,3 | 0,3 | 0,3 | 0,3 |
| Aprikosenkernöl | 0,1 | 0,1 | 0,1 | 0,1 |
| Polyquaternium-10 | 0,5 | 0,5 | 0,5 | 0,5 |
| Cocoamidopropylbetain | 4,0 | 4,0 | 4,0 | 4,0 |
| Calciumsuccinat | --- | --- | --- | 1,3 |
| Calciumlactat | --- | --- | 1,3 | --- |
| Magnesiumcitrat (Trimagnesiumdicitrat) | --- | 1,3 | --- | --- |
| Natriumlactat | 1,3 | --- | --- | --- |
| Milchsäure | ad pH 5,5 | ad pH 4,5 | ad pH 4,5 | ad pH 4,5 |
| Wasser | ad 100 | ad 100 | ad 100 | ad 100 |
| Euperlan PK 3000 AM (1) = (AQUA (WATER), GLYCOL DISTEARATE, GLYCERIN, LAURETH-4, COCAMIDOPROPYL BETAINE, FORMIC ACID (BASF)<br>Croquat WKP PE LQ (2) = Cocodimonium Hydroxypropyl Hydrolyzed Keratin (Croda) | | | | |

3. Messung des Farbschutzes (Rührtest)

Von den Shampoos V1, V2, V3 und E1 wurde jeweils eine 20 %ige wässrige Lösung hergestellt. Diese Lösungen wurden jeweils in ein Becherglas gefüllt. Die colorierten Haarsträhnen wurden jeweils vollständig in eine Lösung eingetaucht. Dann wurde jedes Becherglas auf einem Magnetrührer platziert, ein Rührfisch wurde hineingegeben und es wurde bei 300 U/min gerührt. Nach Ablauf einer Behandlungsdauer von 4 h wurden die Haarsträhnen entnommen, für 60 s mit Leitungswasser (20 °C) ausgespült und trocknen gelassen.

Aus den erhaltenen L*a*b*-Werten wurde der Farbabstand ΔE* zwischen der unbehandelten und der definiert behandelten Strähne gemäß folgender Formel berechnet und aus allen vier Strähnen der Mittelwert gebildet:

$$\Delta E^* = \sqrt{(L_i - L_0)^2 + (a_i - a_0)^2 + (b_i - b_0)^2}$$

$L_i$, $a_i$, $b_i$ = Werte nach Behandlung (Rührtest)
$L_0$, $a_0$, $b_0$ = Werte vor Behandlung (Rührtest)

Für die Bewertungen des Farbschutzes gilt, dass dieser als umso besser eingestuft wird, je kleiner die entsprechenden ΔE*- Werte sind.

| FC1+OX | Shampoo V1 | Shampoo V2 | Shampoo V3 | Shampoo E1 |
|---|---|---|---|---|
| ΔE*-Werte | 15,77 | 14,32 | 12,81 | 12,5 |

| FC2+OX | Shampoo V1 | Shampoo V2 | Shampoo V3 | Shampoo E1 |
|---|---|---|---|---|
| ΔE*-Werte | 15,7 | 12,6 | 12,4 | 12,2 |

4. Messung der Waschechtheit

Zur Bestimmung der Waschbeständigkeit wurde eine 2 %ige Shampoolösung des jeweiligen Shampoos V1, V2, V3 und E1 bis zur oberen Füllmarke in ein Ultraschallbad gefüllt. Darin wurden die colorierten Strähnen vollständig eingetaucht und im Ultraschallbad behandelt (11 min entsprechen 6 Haarwäschen; der Wechsel des Waschwassers erfolgte bei jedem Waschintervall). Pro Nuance und Waschzyklus wurden jeweils drei Tressen direkt nach der Färbung sowie nach 24 Haarwäschen an jeweils vier verschiedenen Punkten (zwei vorne und zwei hinten) farbmetrisch vermessen.

Aus den erhaltenen L*a*b*-Werten wurde der Farbabstand ΔE* zwischen der ungewaschenen und der definiert gewaschenen Strähne gemäß folgender Formel berechnet und aus allen vier Strähnen der Mittelwert gebildet:

$$\Delta E^* = \sqrt{(L_i - L_0)^2 + (a_i - a_0)^2 + (b_i - b_0)^2}$$

$L_i$, $a_i$, $b_i$ = Werte nach 24 Haarwäschen
$L_0$, $a_0$, $b_0$ = Werte nach 0 Haarwäschen

Für die Bewertungen der Waschechtheiten gilt, dass diese als umso schlechter eingestuft werden, je größer die entsprechenden ΔE*- Werte sind.

| FC1+OX, 24 Haarwäschen | Shampoo V1 | Shampoo V2 | Shampoo V3 | Shampoo E1 |
|---|---|---|---|---|
| ΔE*-Werte | 5,3 | 5,7 | 5,4 | 5,1 |

| FC2+OX, 24 Haarwäschen | Shampoo V1 | Shampoo V2 | Shampoo V3 | Shampoo E1 |
|---|---|---|---|---|
| ΔE*-Werte | 5,4 | 4,9 | 4,7 | 4,3 |

5. Messung des Ausblutens

Jedes Shampoo V1, V2, V3 und E1 wurde im Flottenverhältnis 1:5 (1 Teil Shampoo, 5 Teile Haar) auf eine colorierte Haarsträhne appliziert. Die auf diese Weise behandelte Haarsträhne wurde in ein jeweils mit 50 ml Wasser gefülltes Uhrglas gegeben und dort für 10 Minuten belassen. Unter einer Tageslichtlampe wurde die Färbung des Wassers visuell beurteilt (Schulnotensystem, 1 = sehr schwaches Ausbluten, 6 = sehr starkes Ausbluten).

| FC1+OX | Shampoo V1 | Shampoo V2 | Shampoo V3 | Shampoo E1 |
|---|---|---|---|---|
| Ausbluten | 5 | 3 | 3 | 2 |

| FC2+OX | Shampoo V1 | Shampoo V2 | Shampoo V3 | Shampoo E1 |
|---|---|---|---|---|
| Aubluten | 4 | 3 | 4 | 2 |

**Patentansprüche**

1. Haarbehandlungsmittel zur Verringerung und/oder Vermeidung des Ausblutens und/oder Verblassens künstlich erzeugter Haarfarben, welches einen wässrigen kosmetischen Träger umfasst, und

   (a) einen pH-Wert im Bereich von 3,5 bis 6,0 besitzt und
   (b) - bezogen auf sein Gesamtgewicht - 0,4 bis 2,6 Gew.-% Calciumsuccinat enthält.

2. Mittel nach Anspruch 1, **dadurch gekennzeichnet, dass** es (a) einen pH-Wert im Bereich von 3,6 bis 4,9, bevorzugt von 3,8 bis 4,8, weiter bevorzugt von 4,0 bis 4,7 und ganz besonders bevorzugt von 4,2 bis 4,6 besitzt.

3. Mittel nach einem der Ansprüche 1 bis 2, **dadurch gekennzeichnet, dass** es - bezogen auf sein Gesamtgewicht - (b) 0,7 bis 2,3 Gew.-%, bevorzugt 0,9 bis 1,5 Gew.-% und ganz besonders bevorzugt 1,2 bis 1,4 Gew.-% Calciumsuccinat enthält.

4. Mittel nach einem der Ansprüche 1 bis 3, **dadurch gekennzeichnet, dass** es mindestens ein anionisches Tensid aus der Gruppe aus Alkylsulfaten, Alkylethersulfaten, Ethercarbonsäuren und Sulfosuccinaten enthält.

5. Mittel nach einem der Ansprüche 1 bis 4, **dadurch gekennzeichnet, dass** es - bezogen auf sein Gesamtgewicht - ein oder mehrere anionische Tenside aus der Gruppe der Alkylethersulfate in einer Gesamtmenge von 5,5 bis 16,0 Gew.-%, bevorzugt von 6,5 bis 14,0 Gew.-%, weiter bevorzugt von 7,5 bis 12,0 Gew.-% und ganz besonders bevorzugt von 8,0 bis 10,0 Gew.-% enthält.

6. Mittel nach einem der Ansprüche 1 bis 5, **dadurch gekennzeichnet, dass** es mindestens ein amphoteres und/oder zwitterionisches Tensid aus der Gruppe der Betaine, der 2-Alkyl-3-carboxymethyl-3-hydroxyethyl-imidazoline, der N-Alkylglycine, der N-Alkylpropionsäuren, der N-Alkylaminobuttersäuren, der N-Alkyliminodipropionsäuren, der N-Hydroxyethyl-N-alkylamidopropylglycine, der N-Alkyltaurine, der N-Alkylsarcosine, der 2-Alkylaminopropionsäuren und der Alkylaminoessigsäuren, der N-Alkylamphodiacetate und/oder der N-Alkylamphodipropionate enthält.

7. Mittel nach einem der Ansprüche 1 bis 6, **dadurch gekennzeichnet, dass** es - bezogen auf sein Gesamtgewicht - ein oder mehrere zwitterionische Tenside aus der Gruppe der Betaine in einer Gesamtmenge von 1,5 bis 6,5 Gew.-%, bevorzugt von 2,5 bis 5,5 Gew.-%, und besonders bevorzugt von 3,5 bis 4,5 Gew.-% enthält.

8. Verfahren zur Verringerung und/oder Vermeidung des Ausblutens und/oder Verblassens künstlich erzeugter Haarfarben, umfassend die folgenden Schritte in der angegebenen Reihenfolge

   (I) Applizieren eines Färbemittels auf Haare, wobei das Färbemittel mindestens ein Oxidationsfarbstoffvorprodukt und/oder mindestens einen direktziehenden Farbstoff enthält,
   (II) Einwirkenlassen des Färbemittels auf die Haare für einen Zeitraum von 5 Minuten bis 45 Minuten,
   (III) Ausspülen des Färbemittels aus den Haaren,
   (IV) Applizieren eines Farbschutzmittels auf die Haare, wobei es sich bei dem Farbschutzmittel um ein Haarbehandlungsmittel nach einem der Ansprüche 1 bis 7 handelt,
   (V) Einwirkenlassen des Farbschutzmittels für einen Zeitraum von 5 Minuten bis 45 Minuten.

9. Verfahren nach Anspruch 8, **gekennzeichnet durch** das

   (I) Applizieren eines oxidativen Färbmittels auf Haare, wobei das oxidative Färbemittel mindestens ein Oxidationsfarbstoffvorprodukt enthält.

10. Verfahren nach einem der Ansprüche 8 bis 9, **dadurch gekennzeichnet, dass** Schritt (V) gefolgt ist vom
    (VI) Ausspülen des Farbschutzmittels aus den Haaren.

11. Mehrkomponenten-Verpackungseinheit (Kit-of-Parts) zum Behandeln von keratinischen Fasern, insbesondere menschlichen Haaren, umfassend getrennt voneinander konfektioniert

- einen Container (I) enthaltend ein kosmetisches Mittel (A) und
- einen Container (II) enthaltend ein kosmetisches Mittel (B),

wobei

- das Mittel (A) in Container (I) ein Färbemittel ist, das mindestens ein Oxidationsfarbstoffvorprodukt und/oder mindestens einen direktziehenden Farbstoff enthält und
- das Mittel (B) in Container (II) ein Haarbehandlungsmittel nach einem der Ansprüche 1 bis 7 ist.

12. Verwendung eines Haarbehandlungsmittels zum Farbschutz von gefärbten Haaren, wobei das Haarbehandlungsmittel einen wässrigen kosmetischen Träger umfasst, und

(a) einen pH-Wert im Bereich von 3,5 bis 6,0 besitzt und
(b) mindestens ein Erdalkalisalz einer Dicarbonsäure mit 2 bis 8 C-Atomen enthält.

13. Verwendung eines Haarbehandlungsmittels zur Verringerung und/oder Vermeidung des Ausblutens und/oder Verblassens künstlich erzeugter Haarfarben, wobei das Haarbehandlungsmittel einen wässrigen kosmetischen Träger umfasst. und

(a) einen pH-Wert im Bereich von 3,5 bis 6,0 besitzt und
(b) mindestens ein Erdalkalisalz einer Dicarbonsäure mit 2 bis 8 C-Atomen enthält.

14. Verwendung eines Haarbehandlungsmittels nach einem der Ansprüche 12 oder 13, **dadurch gekennzeichnet, dass** das Haarbehandlungsmittel (b) mindestens ein Calciumsalz einer Dicarbonsäure mit 2 bis 8 C-Atomen enthält.

15. Verwendung eines Haarbehandlungsmittels nach einem der Ansprüche 12 bis 14, **dadurch gekennzeichnet, dass** das Haarbehandlungsmittel (b) mindestens ein Erdalkalisalz einer Dicarbonsäure mit 2 bis 8 C-Atomen enthält, das ausgewählt wird aus der Gruppe aus dem Calciumsalz der Bernsteinsäure, dem Magnesiumsalz der Bernsteinsäure, dem Calciumsalz der Malonsäure, dem Magnesiumsalz der Malonsäure, dem Calciumsalz der Maleinsäure, dem Magnesiumsalz der Maleinsäure, dem Calciumsalz der Fumarsäure, dem Magnesiumsalz der Fumarsäure, dem Calciumsalz der Glutaminsäure, dem Magnesiumsalz der Glutaminsäure, dem Calciumsalz der Asparaginsäure, dem Magnesiumsalz der Asparaginsäure, dem Calciumsalz der Oxalsäure, dem Magnesiumsalz der Oxalsäure, dem Calciumsalz der Glutarsäure, dem Magnesiumsalz der Glutarsäure, dem Calciumsalz der Adipinsäure, dem Magnesiumsalz der Adipinsäure, dem Calciumsalz der Pimelinsäure, dem Magnesiumsalz der Pimelinsäure, dem Calciumsalz der Suberinsäure, dem Magnesiumsalz der Suberinsäure, dem Calciumsalz der Tatronsäure, dem Magnesiumsalz der Tatronsäure, dem Calciumsalz der Weinsäure, dem Magnesiumsalz der Weinsäure, dem Calciumsalz der Äpfelsäure, dem Magnesiumsalz der Äpfelsäure, dem Calciumsalz der 2-Oxoglutarsäure, dem Magnesiumsalz der 2-Oxoglutarsäure, dem Calciumsalz der Oxobernsteinsäure und dem Magnesiumsalz der Oxobernsteinsäure.

16. Verwendung eines Haarbehandlungsmittels nach einem der Ansprüche 12 bis 15,**dadurch gekennzeichnet, dass** das Haarbehandlungsmittel - bezogen auf sein Gesamtgewicht - (b) ein oder mehrere Erdalkalisalze von Dicarbonsäuren mit 2 bis 8 C-Atomen in einer Gesamtmenge von 0,1 bis 3,5 Gew.-%, bevorzugt von 0,3 bis 2,8 Gew.-%, weiter bevorzugt von 0,6 bis 2,4 Gew.-% und ganz besonders bevorzugt von 0,8 bis 1,6 Gew.-% enthält.

## Claims

1. Hair treatment composition for reducing and/or preventing bleeding and/or fading of artificially produced hair colours, which comprises an aqueous cosmetic carrier, and

(a) has a pH in the range of 3,5 to 6,0; and
(b) contains - based on its total weight - 0.4 to 2.6% by weight of calcium succinate.

2. Composition according to claim 1, **characterized in that** it has (a) a pH value in the range from 3.6 to 4.9, preferably

from 3.8 to 4.8, more preferably from 4.0 to 4.7 and most preferably from 4.2 to 4.6.

3. Composition according to any one of claims 1 to 2, **characterized in that** it contains - based on its total weight - (b) 0.7 to 2.3% by weight, preferably 0.9 to 1.5% by weight and very particularly preferably 1.2 to 1.4% by weight of calcium succinate.

4. Composition according to any one of claims 1 to 3, **characterized in that** it comprises at least one anionic surfactant selected from the group consisting of alkyl sulphates, alkyl ether sulphates, ether carboxylic acids and sulphosuccinates.

5. Composition according to one of claims 1 to 4, **characterized in that** it contains - based on its total weight - one or more anionic surfactants from the group of alkyl ether sulphates in a total amount of from 5.5 to 16.0% by weight, preferably from 6.5 to 14.0% by weight, more preferably from 7.5 to 12.0% by weight and very preferably from 8.0 to 10.0% by weight.

6. Composition according to any one of claims 1 to 5, **characterized in that** it comprises at least one amphoteric and/or zwitterionic surfactant from the group consisting of the betaines, the 2-alkyl-3-carboxymethyl-3-hydroxyethylimidazolines, the N-alkylglycines, the N-alkylpropionic acids, the N-alkylaminobutyric acids, the N-alkyliminodipropionic acids, the N-hydroxyethyl-N-alkylamidopropylglycines, the N-alkyltaurines, the N-alkylsarcosines, the 2-alkylaminopropionic - acids and the alkylaminoacetic acids, the N-alkylamphodiacetates and/or the N-alkylamphodipropionates.

7. Composition according to one of claims 1 to 6, **characterized in that** it contains - based on its total weight - one or more zwitterionic surfactants from the group of betaines in a total amount of 1.5 to 6.5% by weight, preferably of 2.5 to 5.5% by weight, and particularly preferably of 3.5 to 4.5% by weight.

8. Method for reducing and/or preventing bleeding and/or fading of artificially produced hair dyes, comprising the following steps in the order indicated

   (I) applying a colourant to hair, the colourant comprising at least one oxidation dye precursor and/or at least one direct dye,
   (II) Leave the dye on the hair for a period of 5 minutes to 45 minutes,
   (III) Rinse the dye out of the hair,
   (IV) applying a colour protectant to the hair, wherein the colour protectant is a hair treatment composition according to any one of claims 1 to 7,
   (V) Leave the paint protector to act for a period of 5 minutes to 45 minutes.

9. Method according to claim 8, **characterized by** the

   (I) applying an oxidative colouring agent to hair, wherein the oxidative colouring agent comprises at least one oxidation dye precursor.

10. Method according to any one of claims 8 to 9, **characterized in that** step (V) is followed by the (VI) Rinse the colour protectant out of the hair.

11. Multi-component packaging unit (kit-of-parts) for the treatment of keratinous fibres, in particular human hair, comprising separately made-up

   - a container (I) containing a cosmetic product (A) and
   - a container (II) containing a cosmetic product (B),

   where

   - the agent (A) in container (I) is a colouring agent comprising at least one oxidation dye precursor and/or at least one direct dye, and
   - the agent (B) in container (II) is a hair treatment agent according to any one of claims 1 to 7.

12. Use of a hair treatment composition for colour protection of coloured hair, wherein the hair treatment composition

comprises an aqueous cosmetic carrier, and

(a) has a pH in the range of 3,5 to 6,0; and
(b) contains at least one alkaline earth metal salt of a dicarboxylic acid having 2 to 8 carbon atoms.

**13.** Use of the hair treatment composition for reducing and/or preventing bleeding and/or fading of artificially produced hair colours, wherein the hair treatment composition comprises an aqueous cosmetic carrier, and

(a) has a pH in the range of 3,5 to 6,0; and
(b) contains at least one alkaline earth metal salt of a dicarboxylic acid having 2 to 8 carbon atoms.

**14.** Use of a hair treatment composition according to one of claims 12 or 13, **characterized in that** the hair treatment composition (b) comprises at least one calcium salt of a dicarboxylic acid having 2 to 8 carbon atoms.

**15.** Use of a hair treatment composition according to any one of claims 12 to 14, **characterized in that** the hair treatment composition (b) comprises at least one alkaline earth salt of a dicarboxylic acid having 2 to 8 carbon atoms, which is selected from the group consisting of the calcium salt of succinic acid, the magnesium salt of succinic acid, the calcium salt of malonic acid, the magnesium salt of malonic acid, the calcium salt of maleic acid, the magnesium salt of maleic acid, the calcium salt of fumaric acid, the magnesium salt of fumaric acid, the calcium salt of glutamic acid, the magnesium salt of glutamic acid, the calcium salt of aspartic acid, the magnesium salt of aspartic acid the calcium salt of oxalic acid, the magnesium salt of oxalic acid, the calcium salt of glutaric acid, the magnesium salt of glutaric acid, the calcium salt of adipic acid, the magnesium salt of adipic acid, the calcium salt of pimelic acid, the magnesium salt of pimelic acid, the calcium salt of suberic acid, the magnesium salt of suberic acid, the calcium salt of tartaric acid, the magnesium salt of tartaric acid, the calcium salt of tartaric acid, the magnesium salt of tartaric acid, the calcium salt of malic acid, the magnesium salt of malic acid, the calcium salt of 2-oxoglutaric acid, the magnesium salt of 2-oxoglutaric acid, the calcium salt of oxosuccinic acid and the magnesium salt of oxosuccinic acid.

**16.** Use of a hair treatment composition according to any one of claims 12 to 15,**characterized in that** the hair treatment composition comprises - based on its total weight - (b) one or more alkaline earth metal salts of dicarboxylic acids having 2 to 8 carbon atoms in a total amount of 0.1 to 3.5% by weight, preferably of 0.3 to 2.8% by weight, more preferably of 0.6 to 2.4% by weight and very preferably of 0.8 to 1.6% by weight.

**Revendications**

**1.** Composition de traitement capillaire pour réduire et/ou éviter le dégorgement et/ou la décoloration des colorations capillaires artificielles, qui comprend un véhicule cosmétique aqueux, et

(a) a un pH compris entre 3,5 et 6,0 ; et
(b) - sur la base de son poids total - de 0,4 à 2,6 % en poids de succinate de calcium.

**2.** Agent selon la revendication 1, **caractérisé en ce qu'**il (a) possède une valeur de pH dans la plage de 3,6 à 4,9, de préférence de 3,8 à 4,8, plus préférablement de 4,0 à 4,7 et tout particulièrement de 4,2 à 4,6.

**3.** Agent selon l'une des revendications 1 à 2, **caractérisé en ce qu'**il contient - par rapport à son poids total - (b) 0,7 à 2,3 % en poids, préférentiellement 0,9 à 1,5 % en poids et tout particulièrement 1,2 à 1,4 % en poids de succinate de calcium.

**4.** Produit selon l'une des revendications 1 à 3, **caractérisé en ce qu'**il contient au moins un agent tensioactif anionique choisi dans le groupe constitué par les alkylsulfates, les alkyléthersulfates, les acides éther-carboxyliques et les sulfosuccinates.

**5.** Produit selon l'une des revendications 1 à 4, **caractérisé en ce qu'**il contient - par rapport à son poids total - un ou plusieurs agents tensioactifs anioniques du groupe des alkyléthersulfates en une quantité totale de 5,5 à 16,0 % en poids, de préférence de 6,5 à 14,0 % en poids, de manière encore plus préférée de 7,5 à 12,0 % en poids et de manière tout particulièrement préférée de 8,0 à 10,0 % en poids.

**6.** Agent selon l'une des revendications 1 à 5, **caractérisé en ce qu'**il contient au moins un tensioactif amphotère

et/ou zwitterionique choisi dans le groupe des bétaïnes, des 2-alkyl-3-carboxyméthyl-3-hydroxyéthyl-imidazolines, des N-alkylglycines, des acides N-alkylpropioniques, des acides N-alkylaminobutyriques, des acides N-alkyliminodipropioniques, des N-hydroxyéthyl-N-alkylamidopropylglycines, des N-alkyltaurines, des N-alkylsarcosines, des acides 2-alkylaminopropioniques et des acides alkylaminoacétiques, des N-alkylamphodiacétates et/ou des N-alkylamphodipropionates.

**7.** Agent selon l'une des revendications 1 à 6, **caractérisé en ce qu'**il contient - par rapport à son poids total - un ou plusieurs agents tensioactifs zwitterioniques du groupe des bétaines en une quantité totale de 1,5 à 6,5 % en poids, de préférence de 2,5 à 5,5 % en poids, et de manière particulièrement préférée de 3,5 à 4,5 % en poids.

**8.** Procédé pour réduire et/ou éviter le dégorgement et/ou la décoloration des colorations capillaires obtenues artificiellement, comprenant les étapes suivantes dans l'ordre indiqué

(I) l'application d'un agent de coloration sur les cheveux, l'agent de coloration contenant au moins un précurseur de colorant d'oxydation et/ou au moins un colorant à action directe,
(II) laisser agir le produit de coloration sur les cheveux pendant une période de 5 minutes à 45 minutes
(III) Rincer le produit de coloration des cheveux,
(IV) l'application d'un agent de protection de la couleur sur les cheveux, l'agent de protection de la couleur étant un produit de traitement des cheveux selon l'une quelconque des revendications 1 à 7,
(V) laisser agir le produit de protection des couleurs pendant une période comprise entre 5 minutes et 45 minutes.

**9.** Procédé selon la revendication 8, **caractérisé par le fait que**

(I) l'application d'un colorant d'oxydation sur des cheveux, le colorant d'oxydation contenant au moins un précurseur de colorant d'oxydation.

**10.** Procédé selon l'une quelconque des revendications 8 à 9, **caractérisé en ce que** l'étape (V) est suivie du (VI) Rincer le produit de protection de la couleur des cheveux.

**11.** Unité d'emballage à plusieurs composants (Kit-of-Parts) pour le traitement des fibres kératiniques, en particulier des cheveux humains, comprenant, confectionnés séparément les uns des autres

- un conteneur (I) contenant un produit cosmétique (A) et
- un conteneur (II) contenant un produit cosmétique (B),

où

- l'agent (A) dans le conteneur (I) est un agent colorant qui contient au moins un précurseur de colorant d'oxydation et/ou au moins un colorant direct et
- l'agent (B) dans le conteneur (II) est un agent de traitement capillaire selon l'une des revendications 1 à 7.

**12.** Utilisation d'une composition de traitement capillaire pour la protection de la couleur de cheveux colorés, ladite composition de traitement capillaire comprenant un véhicule cosmétique aqueux, et

(a) a un pH compris entre 3,5 et 6,0 ; et
(b) au moins un sel de métal alcalino-terreux d'un acide dicarboxylique ayant de 2 à 8 atomes de carbone.

**13.** Utilisation de la composition de traitement capillaire pour réduire et/ou éviter le dégorgement et/ou la décoloration de colorations capillaires produites artificiellement, la composition de traitement capillaire comprenant un véhicule cosmétique aqueux, et

(a) a un pH compris entre 3,5 et 6,0 ; et
(b) au moins un sel de métal alcalino-terreux d'un acide dicarboxylique ayant de 2 à 8 atomes de carbone.

**14.** Utilisation d'un produit de traitement capillaire selon l'une des revendications 12 ou 13, **caractérisée en ce que** le produit de traitement capillaire (b) contient au moins un sel de calcium d'un acide dicarboxylique ayant de 2 à 8 atomes de carbone.

**15.** Utilisation d'un produit de traitement capillaire selon l'une des revendications 12 à 14, **caractérisée en ce que** le produit de traitement capillaire (b) contient au moins un sel alcalino-terreux d'un acide dicarboxylique ayant de 2 à 8 atomes de carbone, qui est choisi dans le groupe constitué par le sel de calcium de l'acide succinique, le sel de magnésium de l'acide succinique, le sel de calcium de l'acide malonique, le sel de magnésium de l'acide malonique, le sel de calcium de l'acide maléique, le sel de magnésium de l'acide maléique, le sel de calcium de l'acide fumarique, le sel de magnésium de l'acide fumarique, le sel de calcium de l'acide glutamique, le sel de magnésium de l'acide glutamique, le sel de calcium de l'acide aspartique, le sel de magnésium de l'acide aspartique, le sel de calcium de l'acide oxalique, le sel de magnésium de l'acide oxalique, le sel de calcium de l'acide glutarique, le sel de magnésium de l'acide glutarique, le sel de calcium de l'acide adipique, le sel de magnésium de l'acide adipique, le sel de calcium de l'acide pimélique, le sel de magnésium de l'acide pimélique, le sel de calcium de l'acide subérique, le sel de magnésium de l'acide subérique, le sel de calcium de l'acide tatonique, le sel de magnésium de l'acide tatonique, le sel de calcium de l'acide tartrique, le sel de magnésium de l'acide tartrique, le sel de calcium de l'acide malique, le sel de magnésium de l'acide malique, le sel de calcium de l'acide 2-oxoglutarique, le sel de magnésium de l'acide 2-oxoglutarique, le sel de calcium de l'acide oxosuccinique et le sel de magnésium de l'acide oxosuccinique.

**16.** Utilisation d'un produit de traitement capillaire selon l'une des revendications 12 à 15,**caractérisée en ce que** le produit de traitement capillaire contient - par rapport à son poids total - (b) un ou plusieurs sels alcalino-terreux d'acides dicarboxyliques ayant de 2 à 8 atomes de carbone en une quantité totale de 0,1 à 3,5 % en poids, de préférence de 0,3 à 2,8 % en poids, plus préférablement de 0,6 à 2,4 % en poids et tout particulièrement de 0,8 à 1,6 % en poids.